# EUROPEAN PATENT SPECIFICATION

(11) **EP 3 697 333 B1**
(45) Date of publication and mention of the grant of the patent: **12.01.2022**
(21) Application number: 18808480.0
(22) Date of filing: 16.10.2018
(51) Int. Cl.: A61B 18/20, A61N 5/06

(54) **LASER ENERGY APPLICATOR DEVICE WITH COOLING SYSTEMS**
LASERENERGIEAPPLIKATOR MIT KÜHLSYSTEMEN
DISPOSITIF APPLICATEUR D'ÉNERGIE LASER AVEC SYSTÈMES DE REFROIDISSEMENT

(30) Priority: 18.10.2017 IT 201700117730
(43) Date of publication of application: 26.08.2020
(73) Proprietor: EL.EN. S.p.A., 50041 Calenzano (FI) (IT)
(72) Inventor: MODI, Stefano, 50032 Borgo San Lorenzo (FI) (IT); MASOTTI, Leonardo, 50019 Sesto Fiorentino (FI) (IT)
(74) Representative: Mannucci, Michele
(86) International application number: PCT/IB2018/058012
(87) International publication number: WO 2019/077492

(56) References cited:
- EP-A1- 1 079 896
- US-A1- 2002 035 360
- US-A1- 2002 068 926
- US-A1- 2003 065 314
- US-A1- 2017 165 105

## Description

### TECHNICAL FIELD

The invention relates to devices for the application of electromagnetic energy in the form of a laser beam, particularly for medical use.

### STATE OF THE ART

The use of laser radiation in the medical field is known for various applications. Particularly, in recent years a laser treatment that uses high intensity emission has been developed (High-Intensity Laser Therapy - HILT^{®}), which makes use of pulsed Nd:YAG lasers with peak power intensity of more than 10,000 W/cm². The properties of the laser emission make it possible to obtain a particularly effective treatment in various disorders, thanks to the penetration depth of the beam into the tissues. In fact, many trauma, overload or degenerative disorders involve the deep muscle, tendon and joint structures. By means of HILT^{®} it is possible to reach tissue structures positioned even at considerably depth, even if the light radiation that penetrates the tissues decreases gradually in intensity both as a result of diffusion and of absorption by the tissues.

The new techniques based on HILT^{®} enable considerable depths to be reached without the need to emit excessively high doses of energy, which could damage the more superficial tissues. The pulsed laser used in laser treatment performs various effects on the tissues:
- photochemical effect, which consists of light stimulation of the deep structures and activates cell metabolism and causes activation of fundamental chemical reactions. Transmission of the pain impulse is slowed down, and a rapid analgesic effect is obtained. At cell level, the biostimulating photochemical effect translates into activation of some enzymes, increased production of particular substances (nucleic acids and proteins) and increase of metabolic exchanges, i.e., all the fundamental activities for cell life;
- photomechanical effect, caused by the high peak power of the pulsed laser. The photomechanical effect is due to the generation of elastic pressure waves in the tissues, which propagate in the direction of the pulse that generated them. These waves, propagating inside the tissue, act with a stimulating effect. An effect of stimulation of oxygenation of the tissues, drainage of inflammatory molecules, elimination of fluid collections is obtained, achieving a rapid and intense anti-inflammatory and anti-edema effect and stimulation of the cytoskeleton;
- photothermal effect, which causes a controlled increase of the temperature of the tissues and induces stimulation of the circulation and hyperemia, resulting in an increase in the oxygen supply to the affected structures.

The beneficial effects of the laser, particularly at high intensity, have led to the diffusion of this instrument in the treatment of a plurality of disorders, such as acute and sub-acute muscle lesions, contractures, arthritis and degenerative processes, low back pain, low back pain and sciatica, trochanteric enthesopathy, enthesitis, tendinopathies, tendinitis, epicondylitis, borsitis, synovitis, tenosynovitis, capsulitis, impingement syndrome, post-traumatic or overload disorders, adductor syndromes, distortions, patellar chondropathies, and the like. US 2003/065314 A1 discloses an applicator in accordance to the preamble of claim 1.

Notwithstanding the efficacy shown especially by high intensity laser devices, there is still room for improvement of the results obtainable with the medical treatments using the application of laser energy.

### SUMMARY

The invention is as defined in the appended claims. Embodiments, examples or aspects in the following disclosure, in particular methods, which do not fall under the scope of the claims are presented for illustration purposes only and do not form part of the invention.

According to one aspect, an applicator device is provided, comprising a housing having a mounting interface for a laser emitter and, associated with the housing and connected thereto, a cooling plate with an epidermal contact surface. Moreover, the device is provided with a heat removal system for removing heat from the cooling plate.

In particularly advantageous embodiments, the heat removal system is configured to reach temperatures of the epidermis equal to or higher than 15°C, preferably equal to or higher than 18°C and equal to or lower than 28°C.

To obtain effective heat removal in an applicator device of limited size, easy and practical to use, in some embodiments the heat removal system comprises at least one thermoelectric element, particularly a Peltier cell, in heat exchange relationship with the cooling plate.

To obtain sufficient cooling with devices of small size, it is advantageous, for example, to use a plurality of thermoelectric elements, particularly Peltier cells, in heat exchange relationship with the cooling plate, which can be arranged in series and/or in parallel with one another.

For example, a first thermoelectric element can have a cold face or surface in thermal contact with a rear surface of the cooling plate, and a hot face or surface in thermal contact with a block that acts as heat sink, in turn in thermal contact with the cold surface of one or more thermoelectric elements, preferably in parallel with one another.

In some embodiments, the heat removal system comprises a circuit for the circulation of a refrigerating fluid, for example water, in heat exchange relationship with the cooling plate. The heat exchange relationship between the circuit of the refrigerating fluid and the cooling plate can be a direct heat exchange relationship, i.e., in which the heat flows passively from the cooling plate to the circuit by conduction through materials with sufficient thermal conductivity forming the cooling plate and the circuit of the refrigerating fluid, or part thereof. Masses of conductive material, which increase the thermal inertia of the system, can be inserted between the two components.

Preferably, however, the circuit in which the refrigerating fluid circulates is in an indirect heat exchange relationship with the cooling plate, and one or more thermoelectric elements are arranged between this latter and the circuit of the refrigerating fluid. In this way heat is extracted from the cooling plate by means of thermoelectric effect and heat is removed from the hot surface of the thermoelectric elements by means of the refrigerating fluid circuit.

In practical embodiments, the heat removal system comprises: at least a first thermoelectric element, with a cold surface in contact with the cooling plate and the hot surface in contact with a heat sink; at least a second thermoelectric element, with the cold surface in contact with the heat sink and the hot surface in heat exchange relationship with at least one heat exchange element, in which a refrigerating fluid circulates.

The cooling plate and the laser emitter can be arranged side by side with each other. In other embodiments, the cooling plate has a window or an indentation and the mounting interface of the laser emitter is configured and arranged so that the laser beam emitted by the emitter passes through the cooling plate at the window or indentation. In this way at least a partial overlapping between cooled zone and zone irradiated by the laser beam there can be obtained.

Advantageously, to obtain particularly effective cooling, the window or indentation can be provided with a closing element in a material transparent to the laser radiation and with a high degree of thermal conductivity.

In some embodiments the mounting interface of the laser emitter forms a retention seat, in which the laser emitter can be removably mounted, such that one or another of a plurality of laser emitters can be couple selectively to the applicator device. In other embodiments, the interface can be configured as a stable mounting seat, in which the laser emitter is mounted for example during production and from which it is removed if necessary only in particular circumstances, for example to be replaced or repaired due to faults or for maintenance.

According to another aspect, a laser therapy device is provided, comprising: an applicator device as described above; a laser source; a power source for the system for removing heat from the cooling plate of the applicator device; a laser emitter connectable to the laser source by means of a light guide and applicable to the applicator device.

According to yet another aspect, a method of delivering preferably pulsed laser radiation is described, which does not form part of the invention, comprising the steps of: combining with an emission of laser radiation a removal of heat by means of cooling by contact, for example by means of a cooling plate.

Particularly, disclosed herein is a method, which does not form part of the invention, comprising the steps of: emitting a preferably pulsed laser radiation for a time interval; combining with a laser radiation emission step, a cooling step; particularly combining a plurality of laser radiation emission steps with a plurality of cooling steps. In particular embodiments, the method can include the execution of steps of laser emission alternated with cooling steps without laser radiation.

Further features and embodiments are described in the detailed description of possible embodiments set forth below and in the appended claims, which form an integral part of the present description.

### BRIEF DESCRIPTION OF THE DRAWINGS

The invention will be better understood by following the description and accompanying drawings, which show a non-limiting example of embodiment of the invention. More in particular, in the drawing:
Figs.1A and 1B show schematics of a device according to the present description in two embodiments;
Fig. 2 shows a side view of an applicator device;
Fig.3 shows a view according to III-III of Fig.2;
Fig. 4 shows a partial section according to IV-IV of Fig. 3;
Fig.5 shows an axonometric view of the cooling plate and of components of the respective heat removal system;
Figs. 5A, 5B show sections according to VA-VA of Fig.5B and VB-VB of Fig.5A, respectively;
Fig.6 shows a diagram illustrating the operating mode of the device;
Figs. 7 and 8 show two front views of a cooling plate combined with a laser emitter in other embodiments.

### DETAILED DESCRIPTION OF AN EMBODIMENT

The following detailed description of embodiments given by way of example refers to the accompanying drawings. The same reference numbers in different drawings identify identical or similar elements. Moreover, the drawings are not necessarily to scale. The following detailed description does not limit the invention. Rather, the scope of the invention is defined by the accompanying claims.

Reference in the description to "an embodiment" or "the embodiment" or "some embodiments" means that a particular feature, structure or element described in relation to an embodiment is included in at least one embodiment of the object described. Therefore, the phrase "in an embodiment" or "in the embodiment" or "in some embodiments" used in the description does not necessarily refer to the same embodiment or embodiments. Furthermore, the particular features, structures or elements may be combined in any appropriate manner in one or more embodiments.

Fig.lA shows a schematic of a device 1 according to the present disclosure. The device 1 comprises a housing 2, movable by means of wheels 3 if so required, in which the main components of the device 1 are housed. In the housing 2 a central control unit 5, a laser source 7, a DC electrical energy source 9, a refrigerating circuit 11 are arranged. The central control unit 5 is interfaced with the various components of the device 1, among which particularly the laser source 7, the DC electrical energy source 9, components of the refrigerating circuit 11.

Reference numeral 13 indicates a monitor or a touch screen, and reference numeral 14 indicates one or more user-interfaces, which can comprise a keyboard, a touch pad, a mouse or other element interfacing with the device.

There is associated with the device 1 an applicator device 15, which will be described in more detail below. The applicator device 15 can be connected to components of the device 1 by means of an assembly or bundle of pipes and/or cables indicated as a whole with 17. For example, the applicator device 15, or more precisely a laser emitter associated therewith and described in greater detail hereunder, can be connected to the laser source 7 by means of a fiber optic light guide.

In other embodiments the laser radiation can be guided in an internally hollow articulated arm, provided with mirrors in the joints, which divert the laser beam to the distal end of the arm.

Moreover, data cables and/or electrical power cables can be provided for the various components and functions of the applicator device 15, as better described hereunder. The cables and the light guide can also be combined with delivery and return pipes of a refrigerating fluid, which are part of the refrigerating circuit 11, or are in fluid communication therewith.

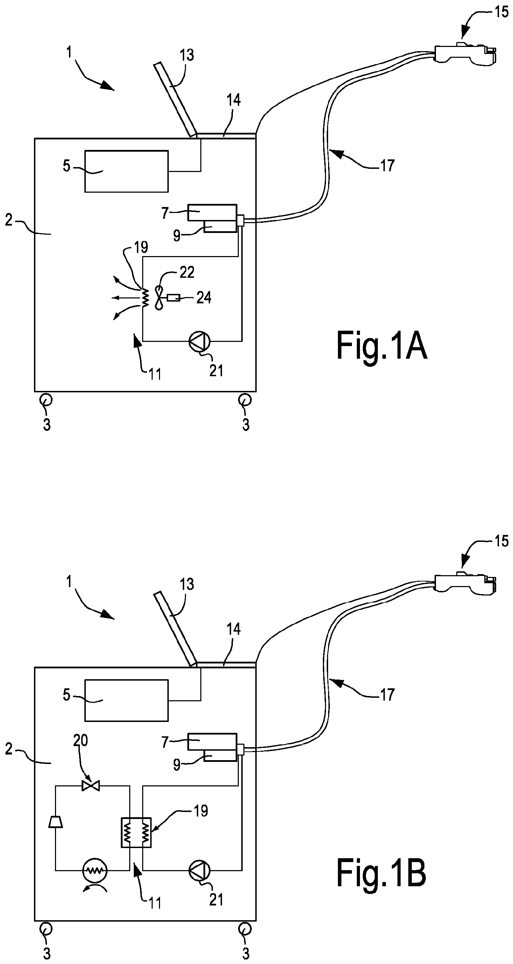

The refrigerating circuit 11 generally comprises heat sink means, schematically shown in Fig. 1A by a heat exchanger 19. The heat exchanger can be a liquid/air exchanger, for example a radiator. The refrigerating fluid can be water, for example distilled water. The heat exchanger 19 can be associated with a fan 22 operated by a motor 24, to obtain a forced convection cooling of the refrigerating fluid that circulates in the refrigerating circuit 11.

The refrigerating fluid circulates in the refrigerating circuit by means of a suitable pumping system, for example comprising a pump 21.

In some cases, if a temperature of the refrigerating fluid lower than ambient temperature is required, for instane, there can be provided a refrigeration system with a refrigeration circuit operated by an electric motor, or an absorption refrigeration system, in both cases configured to cool a flow of refrigerating liquid that circulates toward and from the applicator device 15.

In the embodiment shown schematically in Fig.1B the refrigerating fluid circulates by means of a pump 21 in the pipes connected to the applicator device 15 and exchanges heat in the heat exchanger 19 with a refrigerating fluid circulating in a refrigerating circuit 20. In other embodiments, the refrigerating fluid of the refrigerating circuit 20 can be fed directly to the applicator device 15.

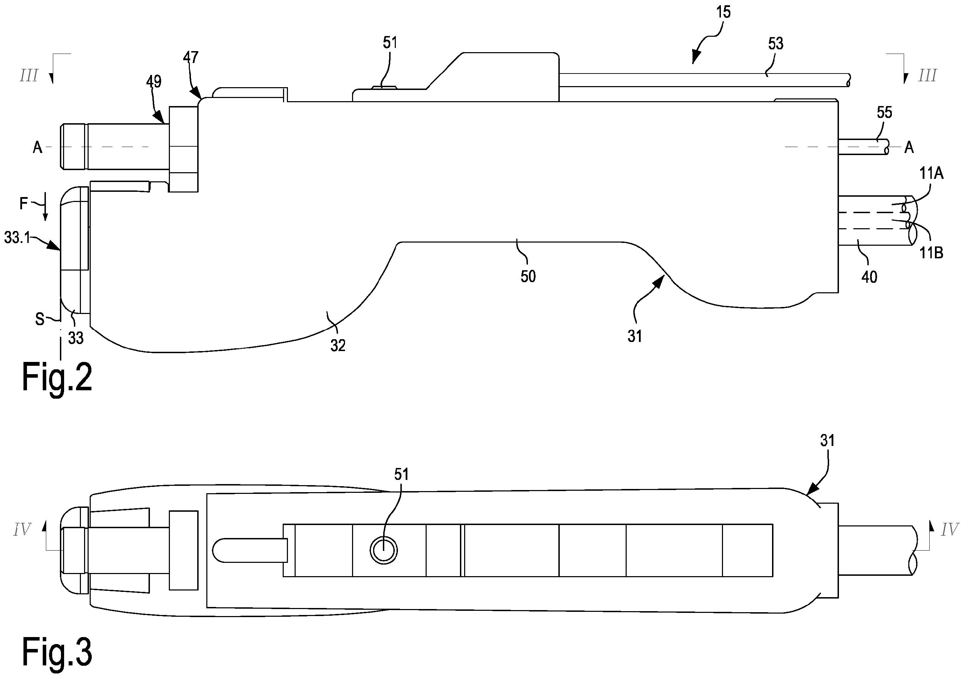

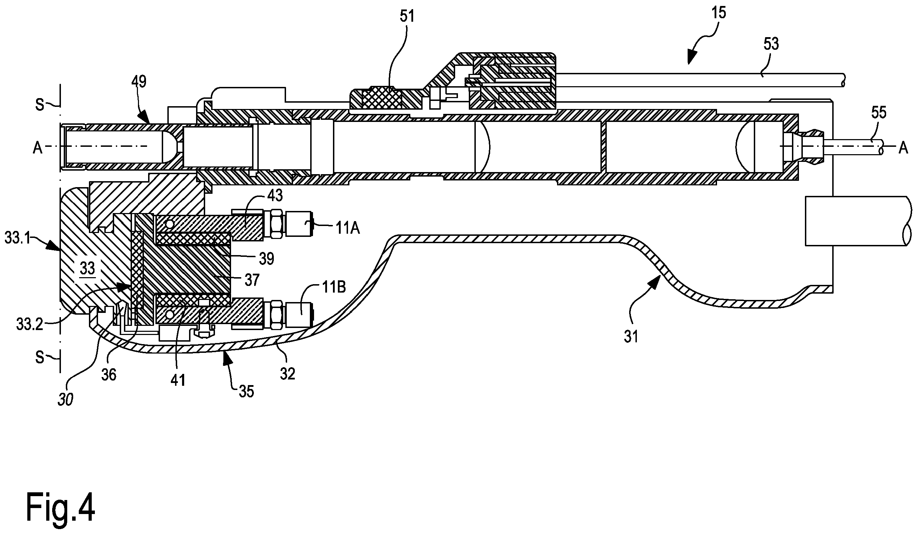

An exemplary embodiment of the applicator device 15 is now described in more detail with reference to Figs. 2 to 5.

The applicator device 15 can comprise a housing 31 that can form a handle and can serve for mounting and/or housing various components of the applicator device 15. The housing 31 can comprise a seat 32, in which a cooling plate 33 is arranged.

The cooling plate 33 can comprise a surface 33.1 outside the seat 32 and a surface 33.2 inside the seat 32. The outer surface 33.1 is an epidermal contact surface. This term means a surface configured to be brought into contact with the epidermis of a patient, to which a laser treatment is applied by means of the applicator device 15.

In the embodiment illustrated, the cooling plate 33 is very thick, i.e. the surfaces 33.1 and 33.2 are at a substantial distance from each other, to give the cooling plate 33 sufficient thermal inertia. For example, the thickness of the cooling plate 33 is of the same order of magnitude as the dimensions of the two surfaces 33.1 and 33.2.

A heat removal system, indicated as a whole with 35, is associated with the cooling plate 33. In some embodiments, the heat removal system 35 comprises one or more Peltier cells, or another element that exploits a thermoelectric effect for cooling. In the embodiment illustrated, the heat removal system 35 comprises a first Peltier cell 36, applied so as to be in thermal contact with the surface 33.2 of the cooling plate 33. The Peltier cell 36 has a cold surface 36F and an opposite hot surface 36C.

"Cold surface" of a Peltier cell is meant herein as the surface that, through thermoelectric effect, is cooled when the Peltier cell is powered. Conversely, "hot surface" of a Peltier cell is meant herein as the surface that, through thermoelectric effect, is heated when the Peltier cell is powered.

The cold surface 36F of the Peltier cell 36 is in thermal contact with the surface 33.2 of the cooling plate 33 and hence in heat exchange relationship with the cooling plate 33. When the Peltier cell is powered, it removes heat from the cooling plate.

The hot surface 36C of the Peltier cell 36 can be in heat exchange relationship with a heat sink, i.e., with a component adapted to absorb heat from the Peltier cell 36. The heat sink can be an air heat sink, such as a finned heat sink, in mechanical contact with the hot surface 36C of the Peltier cell 36 and adapted to dissipate heat in air through natural or forced convection. In other embodiments, the heat sink can be a heat sink in heat exchange relationship with a refrigerating circuit, such as the refrigerating circuit 11.

In the embodiment illustrated, the hot surface 36C of the Peltier cell 36 is in contact with a block 37, made of a material with a high heat exchange coefficient, such as a metal, particularly copper. The block 37 acts as heat sink and absorbs heat emitted by the hot surface 36C of the Peltier cell 36.

In some embodiments, a second Peltier cell or a plurality of second Peltier cells in combination can be associated with the first Peltier cell 36. For example, an arrangement of Peltier cells can be associated in series with the first Peltier cell 36. The arrangement of Peltier cells can comprise one or more Peltier cells in parallel with one another.

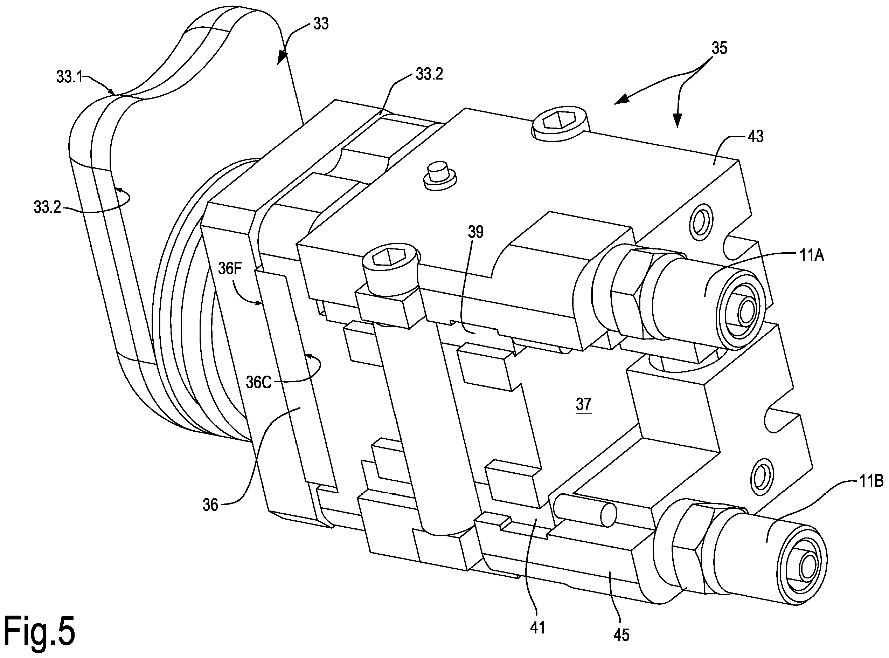

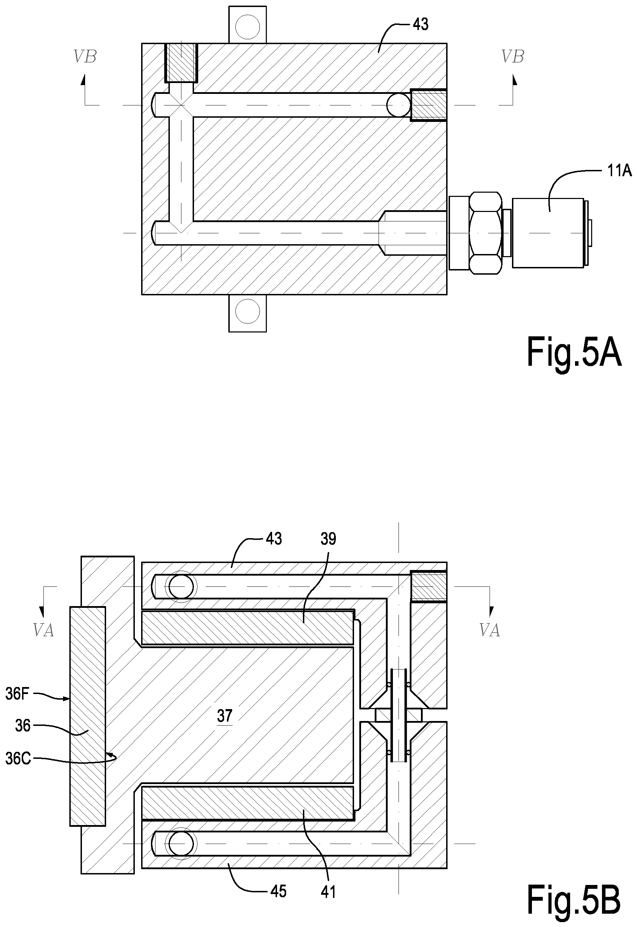

In the exemplary embodiment illustrated (see particularly Fig.5), two further Peltier cells 39, 41, in contact with the block 37 by means of respective cold surfaces, are provided. The hot surfaces of the Peltier cells 39, 41 can be in heat exchange relationship with a heat sink to dissipate heat removed from the block 37. The Peltier cells 39, 41 are therefore arranged in parallel with one another. To dissipate the heat from the hot surface, the two Peltier cells 39, 41 in parallel can be provided with respective finned heat sinks, that dissipate heat in air through forced or natural convection.

In the embodiment illustrated, the hot surfaces of the Peltier cells 39, 41 are in contact with two heat exchange elements 43, 45, connected to each other and respectively with a delivery duct 11A and a return duct 11B of a refrigerating fluid, for example water. When the applicator device (15) is connected to the device 1, the heat exchangers 43, 45 form part of the refrigerating circuit 11, as the two pipes 11A, 11B. Figs. 5A, 5B show in more detail the structure of the heat exchangers 43, 45. Each has a path for the refrigerating fluid. The two paths are indicated with 43A, 45A, respectively, and are connected to each other by a fluid connection 46. The refrigerating fluid is therefore fed by means of the delivery pipe 11A into the heat exchangerg element 43, circulates therein and passes into the heat exchanger element 45, from which it is extracted through the return pipe 11B.

With the arrangement of Figs. 5, 5A, 5B the cooling plate 33 is cooled through thermoelectric effect by the Peltier cell 36, in series with the Peltier cells 39 and 41; the heat produced by these latter is removed by means of the refrigerating fluid that circulates in the pipes 11A, 11B. The combined arrangement of the Peltier cells and of the heat exchanging elements allows high thermal efficiency to be obtained, maintaining a limited difference in temperature between the two opposed surfaces (hot and cold) of each Peltier cell. This condition allows efficient operation of the Peltier cells. Moreover, the configuration is particularly compact and allows it to be produced with a limited size to be housed in the applicator device 15, which must be able to be easily handled by the operator.

In some embodiments the applicator device 15 defines a mounting interface for a laser emitter. In the illustrated exemplary embodiment the interface comprises a seat 47, in which a laser emitter, indicated as a whole with 49, can be housed. The seat 47 can have a longitudinal extension to accommodate the laser emitter 49 in a position such that its optical axis A-A is incident on a flat geometric surface S forming the ideal extension of the epidermal contact surface 33.1.

In other terms, the epidermal contact surface 33.1, which is preferably flat, lies on a geometric surface S. In some embodiments the optical axis A-A of the laser emitter is orthogonal to the geometric surface S when the laser emitter 49 is correctly applied to the mounting interface on the applicator device 15, i.e., when inserted correctly in the seat 47.

The seat 47 is advantageously designed to be able to easily insert and remove one or more different interchangeable laser emitters 49. In some embodiments the seat 47 can be configured to retain the laser emitter 49 by friction, if necessary with the aid of elastic deformation of the walls of the seat 47.

In some embodiments the applicator device 15 has a main body 50, forming a handle, inside which the seat 32 for the heat removal system 35 is arranged, and on the front of which the cooling plate 33 is carried. The seat 47 extends along the longitudinal extension of the main body 50. At the rear end of the handle, opposite the end at which the cooling plate 33 is located, the cables and the pipes that form the assembly or bundle 17, and which will be described in more detail hereunder, are connected. This embodiment of the applicator device 15 is particularly ergonomic and can be easily handled.

In advantageous embodiments, the applicator device 15 is configured so that the distance between the edge of the cooling plate 33 and optical axis A-A of the laser emitter 49 is small, for example equal to or smaller than the diameter of the laser emitter 49. In this way, in use the laser beam emitted by the laser emitter 49 in the direction of the optical axis A-A will impinge on the epidermis of the subject undergoing treatment in a position strictly adjacent to the area involved by the cooling action of the cooling plate 33.

In advantageous embodiments the laser emitter 49 can be interchangeable so that the user can select one or other of a plurality of different laser emitters. For example, the laser emitters 49 can differ in the cross-sectional size or shape of the emitted laser beam, or in the optical characteristics of the laser beam. A laser emitter 49 can, for example, be configured to emit a collimated beam and another laser emitter 49 can be configured to emit a divergent or defocused beam, or to emit a focused beam or a convergent beam.

The laser emitter can comprise, in a manner known *per se,* optical focusing and/or defocusing or collimation systems, which can be adjusted by the user.

In advantageous embodiments, the laser emitter 49 can have a control device 51, for example a control button 51. This control device can be placed in a position to be easily operated by an operator holding the applicator device 15. The control device 51 can be connected by means of a data cable 53 to the central control unit 5 and can be used to control emission of the pulsed laser beam by the laser source 7. In combination or alternatively, the control device can be a pedal element, separate from the applicator device 15.

The laser emitter 49 is connected to the laser source 7 by means of a fiber optic cable 55. Both the fiber optic cable 55 and the data cable 53 can be part of the assembly or bundle 17 of cables and pipes (Fig.1). This assembly or bundle 17 of cables and pipes also includes the delivery and return pipes 11A, 11B of the refrigerating fluid that circulates in the heat exchanger elements 43, 45 of the heat removal system 35. The assembly 17 of cables and pipes can further comprise one or more electrical power cables for the Peltier cells 36, 39, 41, schematically indicated with 40 (Fig.2), which connect the Peltier cells to the DC electrical energy source 9 (Fig. 1).

With the described device 1 and applicator device 15 it is possible to perform laser treatments combined with heat treatments and particularly laser treatments with temperature control of the treated structures. For this purpose, the device 1 is switched on and the heat removal system 35 for removing heat from the cooling plate 33 can be activated. For example, the power supply to one, some or preferably all of the Peltier cells of the heat removal system 35, the circulation of refrigerating fluid in the circuit 11 and the refrigeration circuit 20 can be activated. The laser source 7 is also activated. By means of the user interface 13, 14 it is possible to set various operating parameters. For example, the central control unit 5 can be programmed to provide the operator with the chance to choose between two or more treatment protocols for various areas of the body. In some embodiments, the central control unit 5 can also allow the operator to modify preset operating parameters or define new parameters for different and further treatment protocols, which can then be stored for subsequent use.

Before performing the treatment, the operator can select one of the various laser emitters 49, if available, connect it to the laser source 7 by means of the fiber optic cable 55 and mount it on the applicator device 15.

When the device has been set up and activated, the operator can place the applicator device 15 in contact with the epidermis of a subject to be treated, corresponding to the structures to which laser radiation is to be applied. A non-limiting list of examples of disorders that can be treated effectively with this device is indicated in the introductory part of the present description.

If the heat removal system has been activated, by placing the epidermal contact surface 33.1 of the applicator device 15 in contact with the epidermis of the subject to be treated, the epidermis and the tissues below are cooled. Typically, the temperature of the epidermal contact surface 33.1 is controlled so as to cause a reduction of the temperature of the tissues no lower than 5°C, preferably no lower than 15°C, even more preferably no lower than 18°C and typically between 18°C and 28°C. For this purpose, a temperature sensor 30, adapted to detect the temperature of the cooling plate 33, can be provided. In other embodiments, the temperature of the cooling plate 33 and hence of the epidermis in contact therewith can be estimated, by means of experimental data, collected during calibration, implemented by controlling the current values supplied to the Peltier cells and the temperature and the flow rate of the refrigerating fluid. Cooling can be performed as a preliminary step, before irradiating the area with the laser beam. In some cases, cooling and irradiation treatment can be performed simultaneously.

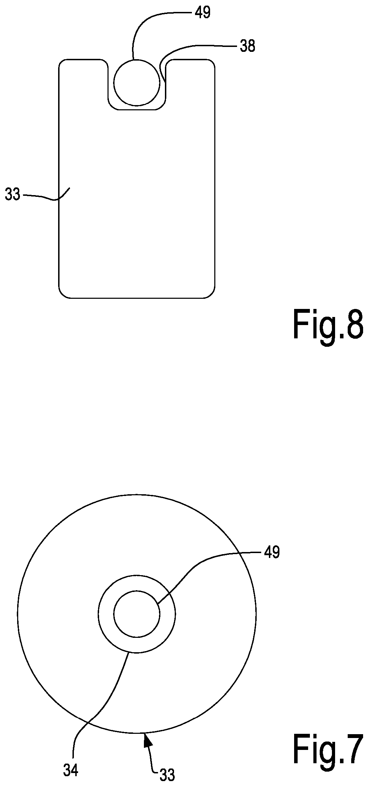

In some variants of embodiment, to facilitate simultaneous treatment, the cooling plate 33 can be shaped so that the laser beam intercepts a portion of epidermis surrounded by an area in direct contact with the cooling plate 33. For example, the cooling plate 33 can be substantially coaxial to the passage of the laser beam and for this purpose have a window or central opening. A possible configuration of this type is represented in Fig. 7, which schematically shows a front view of the cooling plate 33 with a window 34 behind which the laser emitter 49 is installed. The window 34 can be a simple hole in the cooling plate 33. In other embodiments, the window 34 can be closed with a plate in a material transparent to the laser radiation and thermally conductive, such as sapphire.

The window or hole 34 for passage of the laser beam can be central, and therefore roughly coaxial to the cooling plate 33, as shown in Fig. 7. However, this is not essential. In other embodiments, the laser emitter 49 can be positioned offset with respect to the cooling plate 33, until being positioned, for example, in proximity of an edge thereof, as shown in Fig. 8. Here the cooling plate 33 has a lateral indentation 38, behind which the laser emitter 49 is arranged.

In some treatment modes the applicator device can be placed in contact with the epidermis and translated for short distances along the epidermis keeping the epidermal contact surface 33.1 in contact with the epidermis of the patient. The movement can be performed according to the arrow F (Fig.2), so that each portion of epidermis first comes into contact with the epidermal contact surface 33.1 and cooled, and is subsequently irradiated with the laser beam emitted by the laser emitter 49.

The localized cooling of the epidermis and of the tissue below has a double advantageous effect. On the one hand, the temperature reached by the tissues as a result of laser irradiation is reduced and therefore a larger dose of laser energy can be supplied without the risk of thermal damage to the tissues. However, it has been found that, besides this advantage, cooling to a temperature slightly lower than the basal temperature (36°C), creates optimal temperature gradients on the tissues to obtain the maximum synergy of effects with the subsequent laser application, including reactivation of microcirculation at the level of blood and lymphatic capillaries, immediate muscle relaxation capable of alleviating spasms and myofascial pain. Typically, the laser source 7 is a pulsed Nd:YAG laser with a wavelength of 1064 nm.

According to some embodiments, it can be particularly advantageous to apply the laser radiation, preferably pulsed, according to emission intervals distanced in time from one another so as to carry out, between one emission interval and the other, a cooling step of the tissues. For this purpose, the central control unit 5 can be programmed so that, by activating the laser emitter for example by means of the push button 51, the laser source 7 transmits, by means of the light guide, a laser radiation pulsed according to intervals of a predetermined duration and spaced from one another by intervals of time without emission, during which the cooling plate 33 removes heat from the tissues. These intervals without emission represent cooling intervals of the tissues.

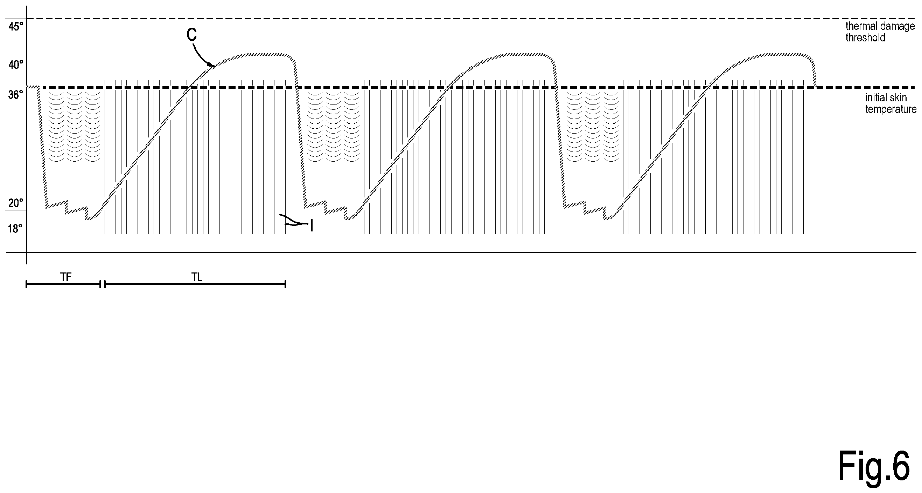

Fig. 6 shows a diagram in which the treatment time is indicated on the abscissa and the temperature of the epidermis is indicated on the ordinate. The curve C indicates the trend of the temperature during treatment. In the diagram provided by way of example in Fig.6 the treatment is performed with cooling time intervals T_{F} of the epidermis by means of the cooling plate 33, alternated with laser treatment time intervals T_{L}. During application of the cooling plate 33 the temperature of the epidermis decreases, for example, to between 20°C and 18°C. In each interval T_{L} subsequent to cooling the laser pulses I cause a gradual increase of the temperature of the epidermis according to the curve C, up to values of around 40°C. These are still below the so-called thermal damage threshold (around 45°C) and therefore the laser radiation does not damage the tissues. After a laser treatment interval T_{L} has terminated, the temperature of the zone treated can be lowered again to around 18-20°C before applying a new series of laser pulses.

The operator can control activation and deactivation of the laser emission by means of the control button 51, or by means of a pedal control (not shown) and can coordinate the movement of the applicator device with activation and deactivation of the laser beam. The control can be such that the pressure on the push button 51 causes start of a laser treatment interval T_{L}, of a duration controlled by the central control unit 5 according to the parameters set by the operator. A subsequent treatment interval T_{L} will be activated only with a subsequent operation of the control button 51. In this way the treatment time T_{L} is controlled accurately by the central control unit 5 and not by the operator.

An addition pedal control with respect to the control button 51 can be used as further safety element. For example, the control button 51 can be enabled only if the pedal control is pressed.

The cooling effect can be suspended simply by moving the cooling plate 33 away from the epidermis.

In some embodiments, the duration of the intervals T_{L} and T_{F} can be fixed, or settable by the operator at the start of treatment and if necessary modifiable during the treatment. In other embodiments, temperature control systems can be provided to modify or modulate the duration of the intervals T_{F} and T_{L}. For example, a temperature sensor can be provided associated with the cooling plate 33.

The duration of the period T_{F} can be extended or reduced based on the speed with which the minimum required temperature, for example 18°C, is reached on the skin.

The times or intervals T_{L} and T_{F} can be set by the control system, or can be set by the operator. In some case, the central control unit 5 can require the operator to set some treatment and/or patient data, based on which T_{L} and T_{F} are calculated automatically. In other cases, the operator can set the T_{L} and T_{F} values at will. Moreover, it would be possible for the T_{L} and T_{F} values to be calculated by the central control unit 5, for example as a function of some or all the operating parameters, and for the operator to be given the chance to modify them, at will and/or within certain limits determined by the central control unit.

The duration of the period T_{L} can be reduced or extended, for example based on the temperature detected on the skin. In some embodiments, the temperature can be detected and the emission of laser radiation can be interrupted upon reaching a maximum set temperature value. Preferably, this control of interruption of the emission is automatic. This makes the treatment particularly efficient and safe, as well as easy to perform. However, it would also be possible to view the temperature on a monitor, a display or another user interface and in this way allow the user to control the emission start and stop times manually.

In some embodiments, one or more temperature sensors can be provided, for example to detect the temperature of the cooling plate and regulate the heat removal system accordingly. In some embodiments, based on a temperature measured, the central control unit 5, or other intelligence installed on the device 1, can modulate the flow rate of the refrigerating fluid in the refrigerating circuit 11 and/or the temperature of the refrigerating fluid. In some embodiments, the central control unit 5, or other intelligence installed on the device 1, can modulate the electrical current supplied to one, to the other, or to all of the Peltier cells 36, 39, 41, if necessary in combination with a modulation of the flow rate of refrigerating fluid and/or of the temperature of the refrigerating fluid in the refrigerating circuit 11.

In advantageous modes of use, the cooling and heating cycles are repeated in sequence, if necessary also in adjacent zones of tissue, to cause alternating phenomena of hyperemia and cooling in the bloodstream, which can be particularly beneficial for the synergy between the cooling effect and laser irradiation.

By way of non-limiting example, the following ranges are indicated for the parameters that can be used in the treatments performed by means of the device 1 and the applicator device 15 described above:
- laser source peak power: 500W - 10000W
- average intensity: 1 - 50W/cm²
- pulse energy: 100-1500 mJ
- average power: 200mW - 100W
- fluence: 100 - 10000 mJ/cm²;
- pulse duration: 5ns-150µs
- pulse repetition frequency: 1-50 Hz

Particularly, with the applicator device and the device described, a treatment method of a patient suffering from one or more of the following disorders can be implemented: painful disorders of the joints, of the tendons, of the ligaments, of the muscles, acute and sub-acute muscle lesions, contractures, arthritis and degenerative processes, low back pain, low back pain and sciatica, trochanteric enthesopathy, enthesitis, tendinopathies, tendinitis, epicondylitis, borsitis, synovitis, tenosynovitis, capsulitis, impingement syndrome, post-traumatic or overload disorders, adductor syndromes, distortions, patellar chondropathies. The method can comprise the following steps:
applying a cooling plate to a portion of epidermis of the patient and removing heat from the epidermis by means of said cooling plate, reducing the temperature of the epidermis and of the tissues below;
applying a laser radiation, preferably pulsed, on the cooled portion of epidermis. The laser radiation is preferably applied in a manner coordinated in time with the cooling.

In embodiments described here, the method can include the step of gradually increasing the temperature of the epidermis. The method can further comprise the step of interrupting application of laser radiation after an application interval, for example after reaching an upper temperature threshold, which can advantageously be lower than the thermal damage threshold, i.e., the threshold above which the temperature reached causes tissue damage. After having reached the upper threshold, a step of temporary interruption of the laser radiation, a cooling step, and a subsequent new irradiation step of with laser radiation can take place. In other embodiments, at least in some intervals the tissue can be simultaneously irradiated through laser radiation and cooled through heat removal by means of the plate of the therapy device.

The method can comprise repetition, at regular and/or modulable and variable intervals, of the laser radiation, causing a cooling of the epidermis between one laser radiation application and the next.

During the laser irradiation step cooling can be interrupted, for example with a step of removing the cooling plate. In other embodiments, cooling can be continuous, for example maintaining a cooling plate in continuous contact with the epidermis, while laser irradiation can be intermittent, with irradiating steps alternated by intervals without irradiation.

The method can comprise the step of moving the cooling plate along the epidermis modifying the contact zone, so that on a given portion of epidermis there are steps of contact with the cooling plate and steps in which the portion of epidermis is not in contact with the cooling plate. The laser beam can be applied to the same zone temporarily in thermal contact with the cooling plate, or can be applied to zones adjacent to the zone in thermal contact with the cooling plate. In some embodiments, the laser beam can, for example, strike a zone of the epidermis surrounded by a zone in contact with the cooling plate. Moreover, the cooling plate can have a window transparent to the laser radiation, to simultaneously have the action of the cooling plate and the action of the laser beam.

The method can comprise the step of cooling the epidermis in the zone of application of the laser beam to temperatures no lower than 5°C and preferably no lower than 10 °C, even more preferably no lower than 15°C, for example no lower than 18°C. Preferably, the temperature to which the epidermis is cooled is no more than 30°C, more preferably no more than 28°C, even more preferably no more than 26°C.

If cooling is not performed continuously, but at intervals, in embodiments of the method described here a laser treatment can be performed until the temperature of the tissues treated increases to values lower than the thermal damage threshold, typically equal to or lower than 40°C, before reactivating the cooling step.

## Claims

1. An applicator device (15) comprising:
- a housing (31) comprising a mounting interface (47) for a laser emitter (49);
- associated with the housing (31) and solidly connected thereto, a cooling plate (33) with an epidermal contact surface (33.1);
- a heat removal system (35) for removing heat from the cooling plate (33); wherein the heat removal system comprises at least a first thermoelectric element (36), with a cold surface (36F) in contact with the cooling plate (33) and a hot surface (36C) in contact with a heat sink (37);
**characterised in that** the heat removal system further comprises at least a second thermoelectric element (39, 41), with a cold surface in contact with the heat sink (37) and a hot surface in a heat exchanging relationship with at least one heat exchange element (43, 45), in which a refrigerating fluid circulates.

2. The applicator device (15) according to claim 1, wherein the first thermoelectric element (36) comprises a Peltier cell in direct or indirect heat exchange relationship with the cooling plate (33) and the second thermoelectric element (39; 41) comprise respective Peltier cell.

3. The applicator device (15) according to claim 1 or 2, wherein the heat removal system (35) comprises a circuit (43, 45) for the circulation of a refrigerating fluid, in heat exchange relationship with the cooling plate (33).

4. The applicator device (15) according to one or more of the previous claims, wherein the mounting interface (47) for the laser emitter (49) and the cooling plate (33) are arranged and configured so that the axis of the laser beam (A-A) emitted by a laser emitter (49) mounted on the interface (47) is incident on a geometric surface constituting an extension of the epidermal contact surface (33.1) of the cooling plate (33).

5. The applicator device (15) according to one or more of the previous claims, comprising a laser emitter (49) coupled with the mounting interface, wherein preferably the laser emitter (49) comprises at least a control device (51) that can be interfaced with a laser source (7), in order to activate or de-activate emission of a laser beam.

6. The applicator device (15) according to one or more of the previous claims, wherein the heat removal system (35) is configured to reach a minimum temperature on the epidermis of no lower than 5°C and preferably no lower than 15°C, and even more preferably no lower than 18°C; and wherein preferably the heat removal system (35) is configured to reach a maximum temperature on the epidermis of no more than 30°C, preferably no more than 28°C, and even more preferably equal to or lower than 26°C.

7. The device according to claim 6, comprising a temperature sensor (30) adapted to detect the temperature of the cooling plate.

8. The applicator device (15) according to one or more of the previous claims, wherein the cooling plate (33) has a window or indentation (34, 38), and wherein the mounting interface (47) for the laser emitter (49) is configured and arranged so that the laser beam emitted by the laser emitter mounted on the interface passes through the cooling plate (33) at the window or indentation (34, 38).

9. The applicator device (15) according to claim 8, wherein the window (34) or cavity (38) is provided with a closing element in a material transparent to the laser radiation and with a high degree of thermal conductivity.

10. The applicator device (15) according to one or more of the previous claims, wherein the mounting interface (47) for the laser emitter (49) forms a seat for stable or removable retention of the laser emitter.

11. A laser therapy device (1), comprising: an applicator device (15) according to one or more of the previous claims; a laser source (7); a power source for the heat removal system (35) for removing heat from the cooling plate (33) of the applicator device (15); a light guide for connecting the laser emitter (49) to the laser source (7).

12. The device according to claim 11, wherein the power source for the heat removal system (35) for removing heat from the cooling plate (33) comprises a source of electrical energy, in particular a DC source (9).

13. The device according to claim 11 or 12, wherein the power source for the heat removal system (35) for removing heat from the cooling plate (33) comprises a refrigerating circuit (11), adapted to circulate a refrigerating fluid, and cooling elements (19; 22; 20) for the refrigerating fluid.

14. The device according to claim 11, 12 or 13, wherein the laser source (7) is a pulsed laser source, particularly with a pulse duration equal to or lower than 200 microseconds, preferably equal to or lower than 150 microseconds, and preferably higher than 5 nanoseconds.

15. The device according to one or more of the previous claims 11 to 14, comprising a control unit (5), particularly adapted to emit a preferably pulsed laser radiation, at repeated emission intervals (TL), spaced in time by non-emission intervals (T_{F}); wherein preferably the control unit (5) is adapted to emit a laser radiation at emission intervals the duration of which is controlled based on a temperature signal and/or based on one or more parameters for laser radiation emission, and wherein the emission parameters for the laser radiation may comprise the following: laser source peak power; maximum intensity; pulse energy; average power; fluency; pulse duration; pulse repetition frequency.

## Patentansprüche

1. Applikatorvorrichtung (15) mit:
- einem Gehäuse (31) mit einer Montagefläche (47) für einen Laseremitter (49),
- der dem Gehäuse (31) zugeordnet ist und fest damit verbunden ist, einer Kühlplatte (33) mit einer Epidermis-Kontaktfläche (33.1),
- einem Wärme-Ableitungssystem (35) zur Ableitung von Wärme von der Kühlplatte (33), wobei das Wärme-Ableitungssystem mindestens ein erstes thermoelektrisches Element (36) mit einer kalten Fläche (36F) in Kontakt mit der Kühlplatte (300) und eine heiße Fläche (36C) in Kontakt mit einer Wärmesenke (37) aufweist,
**dadurch gekennzeichnet, dass** das Wärme-Ableitungssystem ferner mindestens ein zweites thermoelektrisches Element (39, 41) mit einer kalten Fläche in Kontakt mit der Wärmesenke (37) und einer heißen Fläche in einer Wärmetausch-Beziehung mit mindestens einem Wärmetauscher-Element (43, 45) aufweist, in dem ein Kühlfluid zirkuliert.

2. Applikatorvorrichtung (15) nach Anspruch 1, wobei das erste thermoelektrische Element (36) eine Peltier-Zelle in direkter oder indirekter Wärmetauscher-Beziehung mit der Kühlplatte (33) aufweist und das zweite thermoelektrische Element (39; 41) eine jeweilige Peltier-Zelle aufweist.

3. Applikatorvorrichtung (15) nach Anspruch 1 oder 2, wobei das Wärme-Ableitungssystem (35) einen Kreis (43, 45) zur Zirkulation eines Kühlfluids in Wärmetausch-Beziehung mit der Kühlplatte (33) aufweist.

4. Applikatorvorrichtung (15) nach einem oder mehreren der vorstehenden Ansprüche, wobei die Montagefläche (47) für den Laseremitter (49) und die Kühlplatte (33) so angeordnet und ausgebildet sind, dass die Achse des Laserstrahls (A-A), der durch den Laseremitter (49), der auf der Grenzfläche (47) montiert ist, auf eine geometrische Fläche fällt, die eine Verlängerung der epidermischen Kontaktfläche (33. 1) der Kühlplatte (33) bildet.

5. Applikatorvorrichtung (15) nach einem oder mehreren der vorstehenden Ansprüche mit einem Laseremitter (49), der mit der Montagefläche verbunden ist, wobei vorzugsweise der Laseremitter (49) mindestens eine Steuervorrichtung (51) aufweist, die mit einer Laserquelle (7) gekoppelt werden kann, um die Emission eines Laserstrahls zu aktivieren oder zu deaktivieren.

6. Applikatorvorrichtung (15) nach einem oder mehreren der vorstehenden Ansprüche, wobei das Wärme-Ableitungssystem (35) ausgebildet ist, um eine Minimaltemperatur auf der Epidermis von nicht weniger als 5 °C und vorzugsweise nicht weniger als 50 °C und insbesondere vorzuziehen nicht weniger als 18 °C zu erreichen, und wobei vorzugsweise das Wärme-Ableitungssystem (35) ausgebildet ist, um eine Maximaltemperatur auf der Epidermis von nicht mehr als 30 °C, vorzugsweise nicht mehr als 28 °C und insbesondere vorzugsweise gleich oder weniger als 26 °C zu erreichen.

7. Vorrichtung nach Anspruch 6 mit einem Temperatursensor (30), der ausgebildet ist, um die Temperatur der Kühlplatte zu erfassen.

8. Applikatorvorrichtung (15) nach einem oder mehreren der vorstehenden Ansprüche, wobei die Kühlplatte (33) ein Fenster oder eine Einbuchtung (34, 38) aufweist und wobei die Montagefläche (47) für den Laseremitter (49) so ausgebildet und angeordnet ist, dass der Laserstrahl, der durch den Laseremitter, der auf der Fläche montiert ist, emittiert wird, durch die Kühlplatte (33) an dem Fenster oder der Einbuchtung (34, 38) passiert.

9. Applikatorvorrichtung (15) nach Anspruch 8, wobei das Fenster (34) oder ein Hohlraum (38) mit einem Verschlusselement in einem Material, das transparent für die Laserstrahlung ist und einen hohen Grad thermischer Leitfähigkeit hat, versehen ist.

10. Applikatorvorrichtung (15) nach einem oder mehreren der vorstehenden Ansprüche, wobei die Montagefläche (47) für den Laseremitter (49) einen Sitz zur stabilen oder entfernbaren Halterung des Laseremitters bildet.

11. Lasertherapie-Vorrichtung (1) mit: einer Applikatorvorrichtung (15) nach einem oder mehreren der vorstehenden Ansprüche, einer Laserquelle (7), einer Versorgungsquelle für das Wärme-Ableitungssystem (35) zur Ableitung von Wärme von der Kühlplatte (33) der Applikatorvorrichtung (15), einem Lichtleiter zur Verbindung des Laseremitters (49) mit der Laserquelle (7).

12. Vorrichtung nach Anspruch 11, wobei die Versorgungsquelle für das Wärme-Ableitungssystem (35) zur Ableitung von Wärme von der Kühlplatte (33) eine Quelle elektrischer Energie, insbesondere eine Gleichstromquelle (9), aufweist.

13. Vorrichtung nach Anspruch 11 oder 12, wobei die Versorgungsquelle für das Wärme-Ableitungssystem (35) zur Ableitung von Wärme von der Kühlplatte (33) einen Kühlkreis (11), der ausgebildet ist, um ein Kühlfluid zu zirkulieren, und Kühlelemente (19; 22; 20) für das Kühlfluid aufweist.

14. Vorrichtung nach Anspruch 11, 12 oder 13, wobei die Laserquelle (7) eine gepulste Laserquelle ist, vorzugsweise mit einer Pulsdauer gleich oder weniger als 200 µs, vorzugsweise gleich oder weniger als 100 µs und vorzugsweise größer als 5 ns.

15. Vorrichtung nach einem oder mehreren der vorstehenden Ansprüche 11 bis 14 mit einer Steuereinheit (5), die insbesondere ausgebildet ist, um vorzugsweise eine gepulste Laserstrahlung mit sich wiederholenden Emissionsintervallen (TL) zu emittieren, die in der Zeit durch Nicht-Emissionsintervalle beabstandet sind, wobei vorzugsweise die Steuereinheit (5) ausgebildet ist, um eine Laserstrahlung mit Emissionsintervallen zu emittieren, deren Dauer basierend auf einem Temperatursignal und/oder basierend auf einem oder mehreren Parametern für die Laserstrahl-Emission gesteuert sind, und wobei die Emissionsparameter für die Laserstrahlung folgendes aufweisen können: Laserquellen-Spitzenleistung, Maximalintensität, Pulsenergie, mittlere Leistung, Fluss, Pulsdauer, Puls-Wiederholungsfrequenz.

## Revendications

1. Un dispositif applicateur (15) comprenant :
- un logement (31) comprenant une interface de montage (47) pour un émetteur laser (49);
- associée avec le logement (31) et solidaire de celui-ci, une plaque de refroidissement (33) avec une surface de contact épidermique (33.1) ;
- un système d'évacuation de la chaleur (35) pour évacuer de la chaleur de la plaque de refroidissement (33) ;
dans lequel le système d'évacuation de la chaleur comprend au moins un premier élément thermoélectrique (36), avec une surface froide (36F) en contact avec la plaque de refroidissement (33) et une surface chaude (36C) en contact avec un dissipateur thermique (37) ;
**caractérisé en ce que** le système d'évacuation de chaleur comprend en outre au moins un deuxième élément thermoélectrique (39, 41), avec une surface froide en contact avec le dissipateur thermique (37) et une surface chaude en relation d'échange de chaleur avec au moins un élément échangeur de chaleur (43, 45) dans lequel circule un fluide réfrigérant.

2. Le dispositif applicateur (15) selon la revendication 1, dans lequel le premier élément thermoélectrique (36) comprend une cellule Peltier en relation d'échange de chaleur direct ou indirect avec la plaque de refroidissement (33) et le deuxième élément thermoélectrique (39 ; 41) comprend une cellule Peltier correspondante.

3. Le dispositif applicateur (15) selon la revendication 1 ou 2, dans lequel le système d'évacuation de chaleur (35) comprend un circuit (43, 45) pour la circulation d'un fluide réfrigérant, en relation d'échange de chaleur avec la plaque de refroidissement (33).

4. Le dispositif applicateur (15) selon l'une ou plusieurs des revendications précédentes, dans lequel l'interface de montage (47) pour l'émetteur laser (49) et la plaque de refroidissement (33) sont agencées et configurées de telle sorte que l'axe du faisceau laser (A-A) émis par l'émetteur laser (49) monté sur l'interface (47) soit incident sur une surface géométrique constituant une extension de la surface de contact épidermique (33.1) de la plaque de refroidissement (33).

5. Le dispositif applicateur (15) selon l'une ou plusieurs des revendications précédentes, comprenant un émetteur laser (49) couplé à la surface de montage, dans lequel de préférence l'émetteur laser (49) comprend au moins un dispositif de commande (51) qui peut être interfacé avec une source laser (7), afin d'activer ou de désactiver l'émission d'un faisceau laser.

6. Le dispositif applicateur (15) selon l'une ou plusieurs des revendications précédentes, dans lequel le système d'évacuation de chaleur (35) est configuré pour atteindre une température minimale sur l'épiderme d'au moins 5°C et de préférence d'au moins 15°C, et plus préférentiellement encore d'au moins 18°C ; et dans lequel de préférence le système d'évacuation de chaleur (35) est configuré pour atteindre une température maximale sur l'épiderme d'au plus 30°C, de préférence d'au plus 28°C et plus préférentiellement encore inférieure ou égale à 26°C.

7. Le dispositif selon la revendication 6, comprenant un mesureur de température (30) apte à détecter la température de la plaque de refroidissement.

8. Le dispositif applicateur (15) selon l'une ou plusieurs des revendications précédentes, dans lequel la plaque de refroidissement (33) a une fenêtre ou une échancrure (34, 38), et dans lequel l'interface de montage (47) pour l'émetteur laser (49) est configurée et agencée de sorte que le faisceau laser émis par l'émetteur laser monté sur l'interface passe à travers la plaque de refroidissement (33) au niveau de la fenêtre ou de l'échancrure (34, 38).

9. Le dispositif applicateur (15) selon la revendication 8, dans lequel la fenêtre (34) ou la cavité (38) est pourvue d'un élément de fermeture dans un matériau transparent à la radiation laser et avec un degré élevé de conductivité thermique.

10. Le dispositif applicateur (15) selon l'une ou plusieurs des revendications précédentes, dans lequel l'interface de montage (47) pour l'émetteur laser (49) forme un logement pour loger de manière stable ou amovible l'émetteur laser.

11. Un dispositif de thérapie laser (1), comprenant un dispositif applicateur (15) selon l'une ou plusieurs des revendications précédentes ; une source laser (7) ; une source d'énergie pour le système d'évacuation de chaleur (35) pour évacuer de la chaleur de la plaque de refroidissement (33) du dispositif applicateur (15) ; un guide lumineux pour connecter l'émetteur laser (49) à la source laser (7).

12. Le dispositif selon la revendication 11, dans lequel la source d'énergie pour le système d'évacuation de chaleur (35) pour évacuer de la chaleur de la plaque de refroidissement (33) comprend une source d'énergie électrique, en particulier une source de courant continu (9).

13. Le dispositif selon la revendication 11 ou 12, dans lequel la source d'énergie pour le système d'évacuation de chaleur (35) pour évacuer de la chaleur de la plaque de refroidissement (33) comprend un circuit de réfrigération (11), apte à faire circuler un fluide de réfrigération, et des éléments de refroidissement (19 ; 22 ; 20) pour le fluide de réfrigération.

14. Le dispositif selon la revendication 11, 12 ou 13, dans lequel la source laser (7) est une source de laser pulsé, en particulier avec une durée de pulsations égale ou inférieure à 200 microsecondes, de préférence inférieure ou égale à 150 microsecondes et de préférence supérieure à 5 nanosecondes.

15. Le dispositif selon l'une ou plusieurs des revendications 11 à 14, comprenant une unité de commande (5), en particulier apte à émettre une radiation laser de préférence puisée, à intervalles d'émission répétés (TL), espacés dans le temps par des intervalles sans émission (T_{F}) ; dans lequel de préférence l'unité de commande (5) est apte à émettre une radiation laser à des intervalles d'émission dont la durée est commandée sur la base d'un signal de température et/ou sur la base d'un ou de plusieurs paramètres pour l'émission de radiation laser, et dans lequel les paramètres démission pour la radiation laser peuvent comprendre ce qui suit : la puissance de pic de la source laser ; l'intensité maximale ; l'énergie puisée ; la puissance moyenne ; la fluidité ; la durée de pulsations ; la fréquence de répétition des pulsations.
